# EUROPEAN PATENT SPECIFICATION

(11) **EP 2 097 047 B1**
(45) Date of publication and mention of the grant of the patent: **19.05.2010**
(21) Application number: 07859203.7
(22) Date of filing: 21.12.2007
(51) Int. Cl.: A61F 2/46, A61B 17/92

(54) **SURGICAL INSTRUMENT FOR SIMULATING, IN THE INTRAOPERATIVE PHASE, THE FUNCTIONING INSTABILITY OF ACETABULAR COMPONENTS OF HIP PROSTHESES**
CHIRURGISCHES INSTRUMENT ZUR SIMULATION DER FUNKTIONSINSTABILITÄT VON PFANNENKOMPONENTEN EINER HÜFTPROTHESE IN DER INTRAOPERATIVEN PHASE
INSTRUMENT CHIRURGICAL DESTINÉ À SIMULER L'INSTABILITÉ FONCTIONNELLE DE COMPOSANTS ACÉTABULAIRES DE PROTHÈSES DE LA HANCHE AU COURS DE LA PHASE INTRAOPÉRATIVE

(30) Priority: 28.12.2006 IT MI20062533
(43) Date of publication of application: 09.09.2009
(73) Proprietor: Istituto Ortopedico Galeazzi S.P.A., 20161 Milan (IT)
(72) Inventor: GIACOMETTI, Roberto, I-20145 Milan (IT); RAIMONDI, Manuela, Teresa, I-20149 Milano (IT); COLOMBINI, Arianna, I-20010 Mesero (Milan) (IT)
(74) Representative: De Gregori, Antonella
(86) International application number: PCT/IB2007/004128
(87) International publication number: WO 2008/081308

(56) References cited:
- WO-A-2007/070287
- FR-A- 2 776 182
- US-A1- 2004 030 344
- US-B1- 6 395 005

## Description

The present invention relates to the biomedical sector. More specifically, the present invention relates to a surgical instrument for the implanting of hip prostheses, in particular non-cemented acetabular components (metal-backs press-fit inserted in the acetabular cavity).

Hip prostheses are the most widely used joint endoprostheses in reconstructive orthopedic surgery. A hip prosthesis normally consists of a stem made of metal, fixed in the diaphysis channel of the thigh-bone, a femoral head made of metal or ceramics, connected to the stem by means of a conical coupling, an acetabular cup which is articulated on the femoral head, made of UHMWPE (ultra high molecular weight polyethylene) or, more rarely, of ceramics or metal, and a metallic acetabular shell or "metal-back", which rigidly envelops the acetabular cup and which must be inserted in the acetabular cavity of the patent's pelvis.

In order to obtain so-called "press-fit" implants of the acetabular component or shell in the relative cavity, orthopedic surgeons currently use an instrument, called impactor, which allows this acetabular component to be positioned in its cavity and to be implanted by a force of impact.

The clinical duration of an acetabular component implanted in this way or, in other words, not cemented or equipped with transacetabular screws, is strictly linked to the post-operative stability of the implant at the interface with the bone. During the arthroplastic intervention of the hip, in fact, it is difficult to objectively establish the stability of a metal-back press-fit inserted in the acetabular cavity.

Using an impactor of the traditional type, an orthopedic surgeon is capable of implanting an acetabular component relying on his own sensitivity and experience. The tests he effects on the implant are therefore subjective and do not envisage the simulation of a loading condition similar to that in vivo. The necessity is therefore evident of envisaging an instrument which allows the surgeon to reproduce, quantitatively and in the intra-operative phase, the mechanical stress which acts on the metal-back of a hip prosthesis in vivo.

FR 2 776 182 A discloses a surgical instrument for the implantation of acetabular components of hip prostheses comprising a main stem, which acts as a dynamometric impactor for obtaining the implantation of said acetabular component and which comprises at least one dynamometer, coaxial to said main stem, for the application and measuring of a moment on said acetabular component implanted.

An objective of the present invention is to provide a surgical instrument for the implantation of hip prostheses, in particular of non-cemented acetabular components, which envisages the integration, on a single instrument, of both an implantation system and also a system for the simulation of the acetabular biomechanics in the intra-operative phase.

A.further objective of the present invention is to provide a surgical instrument for the implantation of hip prostheses which allows the loading condition which acts in vivo on the implant, to be reproduced, quantitatively and in the intra-operative phase.

Another objective of the present invention is to provide a surgical instrument for the implantation of hip prostheses which can be re-used various times, with the possibility of sterilization in an autoclave, for example.

Yet another objective of the present invention is to provide a surgical instrument for the implantation of hip prostheses which is simple and particularly economical to produce.

These and other objectives according to the present invention are achieved by providing a surgical instrument for the implantation of hip prostheses, in particular non-cemented acetabular components, as specified in claim 1.

Further characteristics of the invention are indicated in the subsequent claims.

The characteristics and advantages of a surgical instrument for the implantation of hip prostheses according to the present invention will appear more evident from the following illustrative and nonlimiting description, referring to the enclosed schematic drawings, in which:

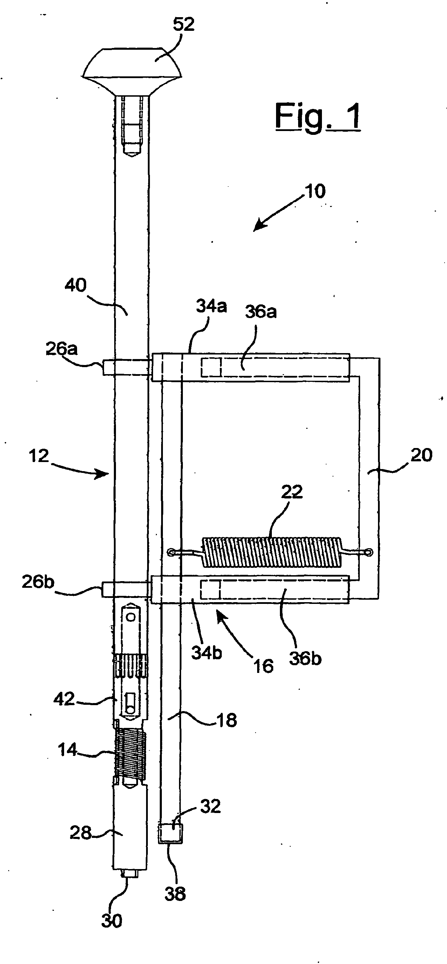

figure 1 is an overall partially sectional view of a surgical instrument for the implantation of hip prostheses according to the present invention;
figure 2 shows two side views, obtained along two different sectional planes, of part of the main stem of the instrument of figure 1;
figure 3 is a raised side view of the impact component of the instrument of figure 1;
figure 4 shows two raised side views, obtained along two different sectional planes, of the central element of the main stem of the instrument of figure 1;
figure 5 is a view from above of the central element shown in figure 4;
figure 6 shows two raised side views, obtained along two different sectional planes, of the lower element of the main stem of the instrument of figure 1;
figure 7 shows two raised side views, obtained along two different sectional planes, of the main stem of the instrument of figure 1, equipped with the impact component of figure 3; and
figure 8 is a raised partially sectional side view of the side mechanism of the instrument of figure 1.

With reference to the figures, these show a surgical instrument for the implanting of hip prostheses according to the present invention, indicated as a whole with the reference number 10.

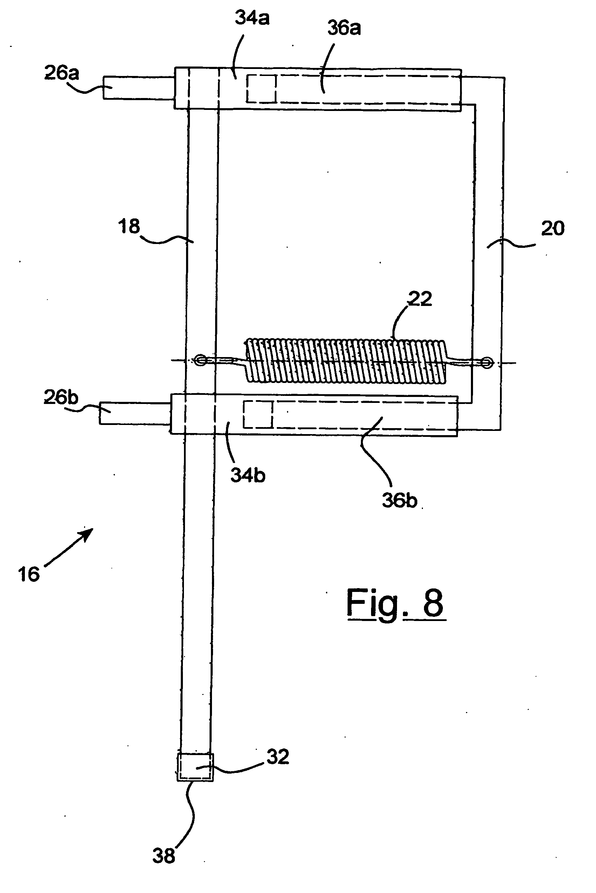

The surgical instrument 10 in its whole, substantially consists of two fundamental parts:
- a main stem 12, which acts as a dynamometric impactor for obtaining the implantation of a generic acetabular component (not shown), comprising at least one torsional dynamometer 14 coaxial thereto for the application and measurement of a torsional moment on the acetabular component implanted, also called metal-back and having the form of a dome or spherical cap; and
- a side mechanism, indicated as a whole with the reference number 16 (figure 8), comprising a secondary stem 18, parallel to the main stem 12, a handle 20 and at least one axial dynamometer 22, orthogonal to the stem 18, for the application and measurement of a destabilizing force on the acetabular component implanted.

The destabilizing force reproduces the action of the articular load, whereas the torsional moment represents the friction moment which acts in vivo. The two stress actions consequently allow the condition in which the patient loads the prosthesis, to be simulated in the intra-operative phase.

The two parts 12 and 16 of the instrument 10 are connected with each other by means of a pair of pass-through cylindrical holes 24a and 24b, situated on the main stem 12 and have an axis perpendicular to the axis of the stem 12 itself. Two pins 26a and 26b obtained on the side mechanism 16 are respectively inserted, by sliding, inside the holes 24a and 24b.

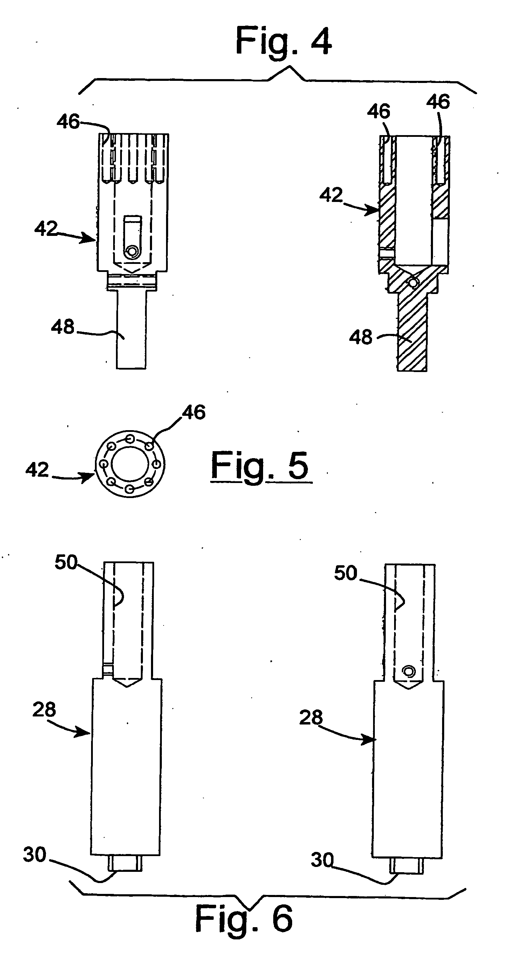

On the free end above the main stem 12, there is an impact component 52, suitable for receiving the impact force to be transmitted to the acetabular component of the prosthesis. In correspondence with the lower free end of the main stem 12, on the other hand, a lower element 28 is applied (figure 6), which can be rotated, equipped with a threaded terminal end 30 for connection with the hole normally situated in correspondence with the top of the metal-back. The secondary stem 18 is in turn equipped with a lower free end 32, preferably covered with a protective hood 38, which, together with the terminal end 30 of the lower element 28, forms the interface system with the metal-back.

The secondary stem 18, connected to the handle 20, is capable of sliding parallel to the main stem 12 until doming to a stop against the internal edge of the metal-back. The adaptability of the interface system with the metal-back of the surgical instrument 10 with acetabular shells having different sizes, is in fact guaranteed by the possibility of sliding the secondary stem 18 with respect to the main stem 12. The only expedient to be adopted relates to the dimensioning of the two stems: the sum of the radius of the main stem 12 and.the diameter of the secondary stem 18 must not be greater than the internal radius of the metal-back.

Surgical compatibility is ensured if the side handle 20 is positioned at a suitable height from the threaded terminal end 30 of the element 28, as the side mechanism 16 must remain external with respect to the patient's pelvis on the operating table.

The secondary stem 18 is made integral with a pair of sleeves 34a and 34b, in turn integral and coaxial with the pins 26a and 26b, respectively. The two end portions 36a and 36b of the side handle 20 are able to slide in the two sleeves 34a and 34b, whereas the axial dynamometer 22, which connects the secondary stem 18 and the side handle 20, consists of a pull spring.

The side handle 20 allows the application of a traction force in a perpendicular direction with respect to the main stem 12 and secondary stem 18. The spring 22 transmits this force to the secondary stem 18 which, in turn, exerts it on the internal edge of the metal-back against which it is buffered by means of its lower end 32. The same spring 22, suitably calibrated, allows the destabilizing force applied on the metal-back to be measured, consequently operating as an axial dynamometer.

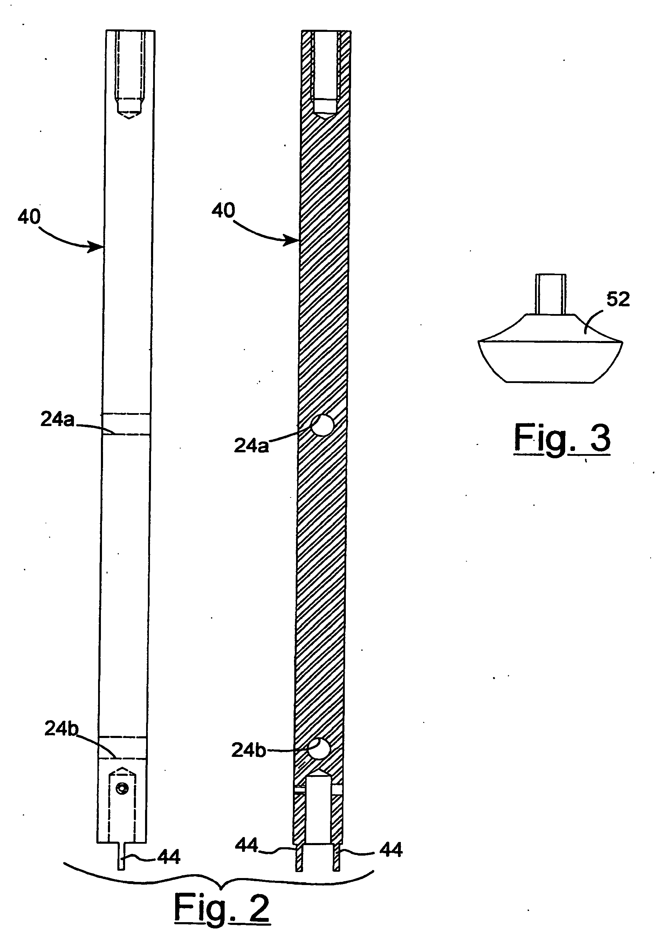

A further system allows the direction of the destabilizing force inside the acetabular cavity to be selected. Between the lower element 28 and the upper tubular body 40 (figure 2), i.e. that in which the side mechanism 16 is inserted, of the main stem 12, there is in fact a central element 42 (figure 4) coaxial to this.

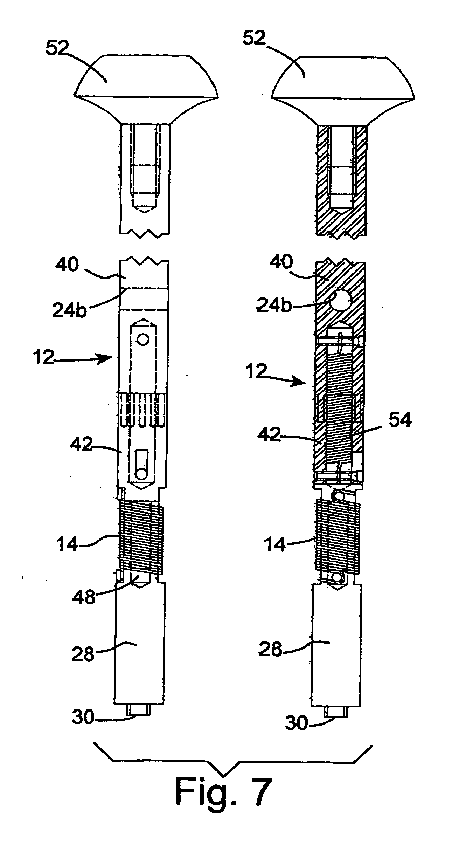

In correspondence with the reciprocal interface portions, the upper body 40 is equipped with a pair of pins 44, whereas the central element 42 has a plurality of axial holes 46 arranged along a circumference. The upper body 40 of the main stem 12 can therefore be press-fit connected with the central element 42 according to various rotation degrees, by insertion of the pins 44 in the relative holes 46, so that the side handle 20 for the application of the force can be positioned in the desired direction with respect to the metal-back. The body 40 and the element 42 are also connected by an internal spring 54 (figure 7), so that the main stem 12 acts as a single piece.

The central element 42 and the lower element 28 of the main stem 12 are connected with each other for example by means of a peg 48, which extends downwards, in an axial direction, from the central element 42 and which is inserted in a corresponding blind hole 50, axially positioned in the lower element 28. The torsional dynamometer 14, which forms the system for the application and measurement of the torsional moment on the implanted metal-back and which is produced, for example, in the form of a torsion spring, is therefore made integral with the central element 42 and lower element 28, in correspondence with their reciprocal contact portions.

The side handle 20 enables the application of a rotation to the upper body 40 of the main stem 12 and the central element 42 connected thereto by insertion, as far as the area in which there is the torsion spring 14. The spring 14 therefore transmits said rotation to the lower element 28 which, in turn, exerts it on the metal-back on which it has been previously screwed. The same torsion spring 14, suitably calibrated, allows the measurement of the torsional moment applied, consequently forming a torsional dynamometer.

The contemporaneousness of the two mechanical actions, i.e. destabilizing force and torsional moment, exerted on the implanted metal-back is guaranteed by the fact that the destabilizing force and the torsional moment are both applied by means of the side handle 20. By acting on the latter, the surgeon can contemporaneously exert a traction on the axial dynamometer 22 and a torsion on the torsional dynamometer 14. The reliability of the measurement of the two mechanical actions on the implanted metal-back is guaranteed by the accuracy of the calibration of the axial 22 and torsional 14 dynamometers.

Sterilizability and surgical safety are ensured by the fact that the surgical instrument 10 according to the present invention can be entirely made of stainless steel, like most of the surgical instruments on the market, or with an analogous hard and shock-resistant material. The possibility of applying the protective hood 38, made with a fine layer of polymeric material, on the free end of the secondary stem 18 allows the internal edge of the metal-back to be protected during the application of the destabilizing force, enabling the transmission of the force but excluding impact between the two metallic parts in contact.

The simplicity of the implantation procedure of the metal-back is maintained due to the fact that the surgical instrument 10 is essentially a variant of an impactor of the traditional type. The surgical time is lengthened only for verifying the stability on the implanted component, which in any case is quite simple and rapid. The simplicity and convenience of use of the surgical instrument 10 are also ensured by the fact that this is essentially a variant of the traditional impactor. The same verification of stability on the implanted component is in any case a simple and intuitive process.

The surgical instrument 10 according to the present invention does not require further instruments for exerting its function as the systems for the application and measurement of the mechanical actions on the implanted metal-back are integrated in the impactor.

The reusability of the surgical instrument 10, in which the pull and torsion springs, suitable calibrated for functioning as dynamometers, can also be made of stainless steel, is guaranteed by its sterilizability.

Finally, the possibility of simulating the mechanical stress actions on the acetabular component allows the implantation process to be verified, providing two important consequences:
- on a clinical level, the surgeon can decide whether or not to use further fixing methods of the acetabular component by means of an objective test, in the intra-operative phase, without having to wait for the result a posteriori of the intervention (which would mean a re-intervention in the case of instability of the implant);
- on a medical-legal level, the surgeon can indicate the verifications of the simulations in the clinical report, as a protection against possible recriminations on the part of the patient.

It can thus be seen that the surgical instrument for the implantation of hip prostheses according to the present invention, achieves the purposes specified above.

The surgical instrument for the implanting of hip prostheses according to the present invention thus conceived can in any case undergo numerous modifications and variants, all included in the same inventive concept; furthermore, all the details can be substituted by technically equivalent elements. In practice, the materials used, as also the forms and dimensions, can vary according to technical requirements.

The protection scope of the invention is therefore defined by the enclosed claims.

## Claims

1. A surgical instrument (10) for the implantation of acetabular components of hip prostheses comprising:
• a main stem (12), which acts as a dynamometric impactor for obtaining the implantation of said acetabular component and which comprises at least one torsional dynamometer (14), coaxial to said main stem (12), for the application and measuring of a torsional moment on said acetabular component implanted; and
• a side mechanism (16), which comprises a secondary stem (18), parallel to said main stem (12), a handle (20) and at least one axial dynamometer 22), orthogonal to said stem (18), which connects said secondary stem (18) to said handle (20) for the application and measuring of a destabilizing force on said acetabular component implanted.

2. The surgical instrument (10) according to claim 1, **characterized in that** said main stem (12) consists of an upper tubular body (40), at least one central element (42) coaxial to said upper tubular body (40), connected thereto by insertion, and at least one lower element (28), coaxial to said upper tubular body (40) and to said central element (42) and which can be rotated with respect to said at least one central element (42) by the interpositioning of said at least one torsional dynamometer (14).

3. The surgical instrument (10) according to claim 2, **characterized in that** said at least one lower element (28) is equipped with a threaded terminal end (30) for connection with said acetabular component.

4. The surgical instrument (10) according to claim 3, **characterized in that** said secondary stem (18) is equipped with a lower free end (32) which, together with said threaded terminal end (30) of said lower element (28), forms the interface system with said acetabular component.

5. The surgical instrument (10) according to claim 4, **characterized in that** said secondary stem (18) is capable of sliding parallel to said main stem (12) to adapt said interface system with said acetabular component to acetabular shells having different dimensions.

6. The surgical instrument (10) according to claim 4, **characterized in that** said lower free end (32) of said secondary stem (18) is covered with a protective hood (38) produced with a thin layer of polymeric material.

7. The surgical instrument (10) according to claim 2, **characterized in that** said side mechanism (16) is connected to said upper body (40) of said main stem (12) by means of one or more pass-through holes (24a, 24b), situated on said upper body (40) and having an axis perpendicular to the axis of said main stem (12), one or more pins (26a, 26b) obtained on said side mechanism (16) being respectively inserted, by sliding, into said holes (24a, 24b).

8. The surgical instrument (10) according to claim 7, **characterized in that** said secondary stem (18) is made integral with one or more sleeves (34a, 34b) in turn integral and coaxial respectively with said one or more pins (26a, 26b).

9. The surgical instrument (10) according to claim 8, **characterized in that** one or more end portions (36a, 36b) of said handle (20) are capable of sliding in said one or more sleeves (34a, 34b), respectively.

10. The surgical instrument (10) according to claim 2, **characterized in that,** in correspondence with the reciprocal interface portions, said upper body (40) is equipped with at least one pin (44) and said central element (42) has a plurality of axial holes (46) arranged along a circumference, said upper body (40) being able to be connected to said central element (42) according to different rotation degrees, by the insertion of said at least one pin (44) in one of said plurality of holes (46), so that said handle (20) can be positioned in the desired direction with respect to said acetabular component.

11. The surgical instrument (10) according to claim 10, **characterized in that** said upper body (40) and said central element (42) are connected by an internal spring (54).

12. The surgical instrument (10) according to claim 2, **characterized in that** said central element (42) and said lower element (28) are connected with each other by means of a peg (48) which extends downwards, in an axial direction, from said central element (42) and which is inserted in a corresponding blind hole (50) positioned axially in said lower element (28).

13. The surgical instrument (10) according to claim 1, **characterized in that** the sum between the radius of said main stem (12) and the diameter of said secondary stem (18) must not be greater than the internal radius of said acetabular component.

14. The surgical instrument (10) according to claim 1, **characterized in that** said torsional dynamometer (14) consists of a torsion spring.

15. The surgical instrument (10) according to claim 1, **characterized in that** said axial dynamometer (22) consists of a pull spring.

16. The surgical instrument (10) according to claim 1, **characterized in that** on the upper free end of said main stem (12) there is an impact component (52) suitable for receiving impact force to be transmitted to said acetabular component.

## Patentansprüche

1. Chirurgisches Instrument (10) für die Implantation von Pfannenkomponenten von Hüftprothesen, umfassend:
einen Hauptschaft (12), der als eine dynamometrische Beaufschlagungseinrichtung zum Erhalten der Implantation der Pfannenkomponente wirkt und der zumindest einen Torsionsdynamometer (14) koaxial zu dem Hauptschaft (12) für das Aufbringen und Messen eines Torsionsmomentes auf die implantierte Pfannenkomponente umfasst; und
einen Seitenmechanismus (16), der einen sekundären Schaft (18) parallel zu dem Hauptschaft (12), einen Griff (20) und zumindest einen axialen Dynamometer (22) orthogonal zu dem Schaft (18) umfasst und der den sekundären Schaft (18) mit dem Griff (20) für das Aufbringen und Messen einer destabilisierenden Kraft auf die implantierte Pfannenkomponente verbindet.

2. Chirurgisches Instrument (10) nach Anspruch 1,
**dadurch gekennzeichnet, dass**
der Hauptschaft (12) aus einem oberen rohrförmigen Körper (40), zumindest einem Zentralelement (42) koaxial zu dem oberen rohrförmigen Körper (40), das daran durch Einsetzen verbunden ist, und zumindest ein unteres Element (28) koaxial zu dem oberen rohrförmigen Körper (40) und zu dem Zentralelement (42) besteht, und das in Bezug auf das zumindest eine Zentralelement (42) **dadurch** gedreht werden kann, dass der zumindest eine Torsionsdynamometer (14) dazwischen angeordnet ist.

3. Chirurgisches Instrument (10) nach Anspruch 2,
**dadurch gekennzeichnet, dass**
das zumindest eine untere Element (28) mit einem Gewindeanschlussende (30) zur Verbindung mit der Pfannenkomponente ausgestattet ist.

4. Chirurgisches Instrument (10) nach Anspruch 3,
**dadurch gekennzeichnet, dass**
der sekundäre Schaft (18) mit einem unteren freien Ende (32) ausgestattet ist, das zusammen mit dem Gewindeanschlussende (30) des unteren Elementes (28) das Schnittstellensystem mit der Pfannenkomponente bildet.

5. Chirurgisches Instrument (10) nach Anspruch 4,
**dadurch gekennzeichnet, dass**
der sekundäre Schaft (18) in der Lage ist, parallel zu dem Hauptschaft (12) zu gleiten, um das Schnittstellensystem mit der Pfannenkomponente an Pfannenschalen mit unterschiedlichen Abmessungen anzupassen.

6. Chirurgisches Instrument (10) nach Anspruch 4,
**dadurch gekennzeichnet, dass**
das untere freie Ende (32) des sekundären Schaftes (18) mit einer Schutzhaube (38) bedeckt ist, die mit einer dünnen Schicht aus Polymermaterial hergestellt ist.

7. Chirurgisches Instrument (10) nach Anspruch 2,
**dadurch gekennzeichnet, dass**
der Seitenmechanismus (16) mit dem oberen Körper (40) des Hauptschaftes (12) mittels von einem oder mehreren Durchgangslöchern (24a, 24b) verbunden ist, die an dem oberen Körper (40) angeordnet sind und eine Achse aufweisen, die rechtwinklig zu der Achse des Hauptschaftes (12) ist, wobei ein oder mehrere Stifte (26a, 26b), die an dem Seitenmechanismus (16) enthalten sind, jeweils durch Gleiten in die Löcher (24a, 24b) eingesetzt sind.

8. Chirurgisches Instrument (10) nach Anspruch 7,
**dadurch gekennzeichnet, dass**
der sekundäre Schaft (18) einteilig mit einer oder mehreren Hülsen (34a, 34b) ausgebildet ist, die ihrerseits einteilig und koaxial jeweils mit dem einen oder den mehreren Stiften (26a, 26b) sind.

9. Chirurgisches Instrument (10) nach Anspruch 8,
**dadurch gekennzeichnet, dass**
ein oder mehrere Endabschnitte (36a, 36b) des Griffes (20) in der Lage sind, in der einen bzw. den mehreren Hülsen (34a, 34b) zu gleiten.

10. Chirurgisches Instrument (10) nach Anspruch 2,
**dadurch gekennzeichnet, dass**
in Übereinstimmung mit den reziproken Schnittstellenabschnitten der obere Körper (40) mit zumindest einem Stift (44) ausgestattet ist und das Zentralelement (42) eine Vielzahl axialer Löcher (46) aufweist, die entlang einem Umfang angeordnet sind, wobei der obere Körper (40) in der Lage ist, mit dem Zentralelement (42) gemäß unterschiedlicher Rotationsgrade durch das Einsetzen des zumindest einen Stiftes (44) in eines der Vielzahl von Löchern (46) verbunden zu werden, so dass der Griff (20) in der gewünschten Richtung in Bezug auf die Pfannenkomponente positioniert werden kann.

11. Chirurgisches Instrument (10) nach Anspruch 10,
**dadurch gekennzeichnet, dass**
der obere Körper (40) und das Zentralelement (42) durch eine innere Feder (54) verbunden sind.

12. Chirurgisches Instrument (10) nach Anspruch 2,
**dadurch gekennzeichnet, dass**
das Zentralelement (42) und das untere Element (28) miteinander mittels eines Zapfens (48) verbunden sind, der sich in einer axialen Richtung von dem Zentralelement (42) abwärts erstreckt und der in ein entsprechendes Blindloch (50) eingesetzt ist, das axial in dem unteren Element (28) positioniert ist.

13. Chirurgisches Instrument (10) nach Anspruch 1,
**dadurch gekennzeichnet, dass**
die Summe zwischen dem Radius des Hauptschaftes (12) und dem Durchmesser des sekundären Schaftes (18) nicht größer als der Innenradius der Pfannenkomponente sein darf.

14. Chirurgisches Instrument (10) nach Anspruch 1,
**dadurch gekennzeichnet, dass**
der Torsionsdynamometer (14) aus einer Torsionsfeder besteht.

15. Chirurgisches Instrument (10) nach Anspruch 1,
**dadurch gekennzeichnet, dass**
der axiale Dynamometer (22) aus einer Zugfeder besteht.

16. Chirurgisches Instrument (10) nach Anspruch 1,
**dadurch gekennzeichnet, dass**
sich an dem oberen freien Ende des Hauptschaftes (12) eine Beaufschlagungskomponente (52) befindet, die zur Aufnahme einer Beaufschlagungskraft geeignet ist, die an die Pfannenkomponente übertragen werden soll.

## Revendications

1. Instrument chirurgical (10) pour l'implantation de composants acétabulaires de prothèses de hanche, comprenant :
- une tige principale (12), qui agit comme un impacteur dynamométrique pour obtenir l'implantation dudit composant acétabulaire et qui comprend au moins un dynamomètre de torsion (14), coaxial à ladite tige principale (12), pour l'application et la mesure d'un moment de torsion sur ledit composant acétabulaire implanté, et
- un mécanisme latéral (16) qui comprend une tige secondaire (18), parallèle à ladite tige principale (12), une poignée (20) et au moins un dynamomètre axial (22), perpendiculaire à ladite tige (18), qui relie ladite tige secondaire (18) à ladite poignée (20), pour l'application et la mesure d'une force de déstabilisation sur ledit composant acétabulaire implanté.

2. Instrument chirurgical (10) selon la revendication 1, **caractérisé en ce que** ladite tige principale (12) est constituée d'un corps tubulaire supérieur (40), d'au moins un élément central (42) coaxial audit corps tubulaire supérieur (40), connecté à celui-ci par insertion, et d'au moins un élément inférieur (28), coaxial audit corps tubulaire supérieur (40) et audit élément central (42) et qui peut être tourné relativement audit au moins un élément central (42) par l'interposition dudit au moins un dynamomètre de torsion (14).

3. Instrument chirurgical (10) selon la revendication 2, **caractérisé en ce que** ledit au moins un élément inférieur (28) est équipé d'une extrémité terminale filetée (30) pour connexion avec ledit composant acétabulaire.

4. Instrument chirurgical (10) selon la revendication 3, **caractérisé en ce que** ladite tige secondaire (18) est équipée d'une extrémité libre inférieure (32) qui, avec ladite extrémité terminale filetée (30) dudit élément inférieur (28), forme le système d'interface avec ledit composant acétabulaire.

5. Instrument chirurgical (10) selon la revendication 4, **caractérisé en ce que** ladite tige secondaire (18) est capable de coulisser parallèlement à ladite tige principale (12) pour adapter ledit système d'interface avec ledit composant acétabulaire aux coques acétabulaires ayant des dimensions différentes.

6. Instrument chirurgical (10) selon la revendication 4, **caractérisé en ce que** ladite extrémité libre inférieure (32) de ladite tige secondaire (18) est recouverte d'un capot de protection (38) produit avec une couche mince de matériau polymère.

7. Instrument chirurgical (10) selon la revendication 2, **caractérisé en ce que** ledit mécanisme latéral (16) est connecté audit corps supérieur (40) de ladite tige principale (12), au moyen d'un ou plusieurs orifices traversants (24a, 24b) situés sur ledit corps supérieur (40) et ayant un axe perpendiculaire à l'axe de ladite tige principale (12), une ou plusieurs broches (26a, 26b) obtenues sur ledit mécanisme latéral (16) étant respectivement insérées, par coulissement, dans lesdits orifices (24a, 24b).

8. Instrument chirurgical (10) selon la revendication 7, **caractérisé en ce que** ladite tige secondaire (18) est réalisée d'un seul tenant avec un ou plusieurs manchons (34a, 34b) à leur tour d'un seul tenant et coaxiaux respectivement avec ladite ou lesdites broches (26a, 26b).

9. Instrument chirurgical (10) selon la revendication 8, **caractérisé en ce qu'**une ou plusieurs portions finales (36a, 36b) de ladite poignée (20) sont capables de coulisser dans ledit ou lesdits manchons (34a, 34b) respectivement.

10. Instrument chirurgical (10) selon la revendication 2, **caractérisé en ce que,** au niveau des portions d'interface réciproques, ledit corps supérieur (40) est équipé d'au moins une broche (44) et ledit élément central (42) a une pluralité de trous axiaux (46) disposés le long d'une circonférence, ledit corps supérieur (40) étant capable d'être connecté audit élément central (42) selon différents degrés de rotation, par l'insertion de ladite au moins une broche (44) dans l'un parmi la pluralité de trous (46), de sorte que ladite poignée (20) puisse être positionnée dans la direction souhaitée relativement audit composant acétabulaire.

11. Instrument chirurgical (10) selon la revendication 10, **caractérisé en ce que** ledit corps supérieur (40) et ledit élément central (42) sont raccordés par un ressort interne (54).

12. Instrument chirurgical (10) selon la revendication 2, **caractérisé en ce que** ledit élément central (42) et ledit élément inférieur (28) sont connectés l'un avec l'autre au moyen d'une cheville (48) qui s'étend vers le bas, dans une direction axiale, depuis ledit élément central (42) et qui est insérée dans un orifice borgne correspondant (50) positionné axialement dans ledit élément inférieur (28).

13. Instrument chirurgical (10) selon la revendication 1, **caractérisé en ce que** la somme entre le rayon de ladite tige principale (12) et le diamètre de ladite tige secondaire (18) ne doit pas être supérieure au rayon interne dudit composant acétabulaire.

14. Instrument chirurgical (10) selon la revendication 1, **caractérisé en ce que** ledit dynamomètre de torsion (14) est constitué d'un ressort de torsion.

15. Instrument chirurgical (10) selon la revendication 1, **caractérisé en ce que** ledit dynamomètre axial (22) est constitué d'un ressort de traction.

16. Instrument chirurgical (10) selon la revendication 1, **caractérisé en ce que,** sur ladite extrémité libre supérieure de ladite tige principale (12), il existe un composant d'impact (52) adapté pour recevoir la force d'impact à transmettre audit composant acétabulaire.
